# EUROPEAN PATENT SPECIFICATION

(11) **EP 1 315 444 B1**
(45) Date of publication and mention of the grant of the patent: **03.05.2006**
(21) Application number: 01963257.9
(22) Date of filing: 05.09.2001
(51) Int. Cl.: A61B 1/01

(54) **BODY CAVITY LINER**
KÖRPERHOHLRAUMVERKLEIDUNG
MANCHON DESTINE A UNE CAVITE CORPORELLE

(30) Priority: 05.09.2000 GB 0021775; 17.11.2000 GB 0028131; 29.08.2001 GB 0120934
(43) Date of publication of application: 04.06.2003
(73) Proprietor: Medevert Limited, Lymington Hampshire SO41 9BE (GB)
(72) Inventor: POOLE, Anthony, George, Fordingbridge, Hampshire SP6 2PT (GB); YOUNG, Antony, John, London WIT 3LU (GB)
(74) Representative: Foster, Mark Charles
(86) International application number: PCT/GB2001/003998
(87) International publication number: WO 2002/019899

(56) References cited:
- WO-A-85/00097
- US-A- 5 236 423
- US-A- 5 458 573
- US-A- 6 004 340

## Description

The present invention relates to a body cavity liner and a method of lining a body cavity of an inanimate object, including an excised or artificial human or animal body part.

Colon cancer is a common form of cancer. Evidence suggests that the majority of colonic malignancy originates in previously benign polyps, and most colon cancer could be prevented if these polyps were detected and removed while still benign.

Colonoscopy is a commonly used diagnostic procedure when malignancy of the large intestine is suspected. In this procedure the interior of the colon is examined using an elongated flexible fibre optic endoscope.

The endoscope used in transanal colonoscopy includes a flexible tube sufficiently long that it can extend from the anal canal through the full length of the colon so that its inner end reaches the caecum. Typically an endoscope is 1.8 metres long and approximately 1 centimetre in diameter. The endoscope is sufficiently stiff so that it does not buckle when it is inserted. The final six inches of the inward (in use) end of the endoscope is usually manoeuvrable by operating controls at the end of the endoscope outside the patient. The inward tip of the endoscope typically incorporates a light source and fibre optics for illumination and visual observation, and tools for performing irrigation, suction and surgical procedures such as polyp removal.

The most commonly used procedure for examining the colon is first to insert the endoscope as far as is desired while inspecting as the endoscope advances. A detailed examination of the colon is made as the endoscope is withdrawn.

To examine the entire colon, the endoscope is inserted through the anal opening and anal canal into the rectum, and then advanced through the sigmoid flexure into the descending colon. The endoscope then passes through the left colic flexure (the splenic flexure) into the transverse colon, and then through the right coiic flexure (the hepatic flexure). The endoscope next passes through the ascending colon and finally reaches the caecum.

To insert the endoscope the device is grasped at a point outside the body near the anal opening. The operator pushes the endoscope inwards and also manipulates the endoscope so that the inward tip is aimed in the required direction. Advancing the endoscope within the colon is a difficult procedure. It is particularly difficult to advance the endoscope through the sharp bends of the colon at the sigmoid flexure and the splenic flexure. When the endoscope passes through these bends the colon can distend and the pressure of the endoscope on the colon walls will tend to stretch that portion of the colon through which the endoscope has already passed, rather than advancing the tip further into the colon. If the colon becomes Irritated by the movement of the colonoscope within it, a reflex action of the colon wall can cause the colon to constrict around the endoscope, increasing the tendency of the colon to distend lengthwise. This reflex action is caused by the circumferential colonic musculature. Muscle relaxants can be given to the patient being examined to relax the circumferential colonic musculature; however, these relaxants will also relax the longitudinal colonic musculature, which will cause still further lengthwise stretching of the colon, inhibiting advancement of the endoscope tip.

The insertion of the endoscope can be uncomfortable for the patient. However, it is generally undesirable to anaesthetise the patient because the patient will be unable to provide feedback to the person inserting the endoscope regarding the presence or intensity of pain, which will provide the inserter with a helpful indication that the endoscope has been misdirected.

There is a substantial risk of perforation of the colon by the tip of the endoscope during insertion, even when performed by skilled and experienced surgeons.

Although the inspection of the colon itself, is mainly performed during withdrawal of the endoscope, which typically takes approximately 10 minutes, insertion of the endoscope can take as much as 50 minutes. Too often the insertion of the endoscope is halted when the tip is at a point short of the caecum because either the patient complains of increasing discomfort and pain, which indicates the bowel mesentry is being dangerously stretched or looping of the bowel has occurred, i.e. the bowel lengthens without the tip of the endoscope advancing. For example, at the sigmoid flexure or the transverse colon, these problems can occur and prevent further advance. This leaves diagnosis incomplete because a portion of the colon beyond the tip of the endoscope will not have been examined.

Another problem with conventional endoscopic advancing devices is that they tend to stretch the colon when they reach a corner. This can result in a colon looping around itself. Looping occurs as a result of applying forward pressure to an endoscope when the required direction of movement of the endoscope is different to the force applied. In the prior art, using the endoscope controls the distal end is manipulated to form a hook. The endoscope is then pulled backwards, which will, under control of a skilled clinician, straighten the colon and allow further advancement subsequently.

It is known to evert a flexible liner into the colon. The endoscope can then be inserted into the colon within the liner. US Patent No. 5236423 describes the eversion of a liner into a body cavity and the subsequent insertion of an endoscope within the liner. Generally such liners comprise a single walled tube of flexible material. Opposite ends of the liner are secured in position and fluid is pumped between these opposite ends to cause the liner to evert.

Prior art eversion liners require a pressure of about 3 bar to advance within the colon. This is a dangerously high pressure for the patient. Generally, the pressure required to hold the endoscope in position is far less than required for advancing (approximately 0.5 bar as opposed to 3 bar).

US-A-5458573 discloses a toposcopic dilatation catheter system utilizing a dilatation balloon in combination with an everting tube in a miniature catheter of a scale sufficiently small to negotiate a blood vessel for therapeutic as well as diagnostic purposes. The catheter system utilizes a primary catheter shaft provided with multiple lumen or passageways extending the length thereof. The dilatation balloon lumen provides access to a balloon carried at the distal end of the primary catheter shaft. The dilatation balloon may be expanded as required to aid in advancing the catheter system to the site requiring therapy. A secondary catheter tube is coaxially carried within the primary catheter shaft The leading end of the secondary catheter tube includes an everting tube which everts from the leading end of the primary catheter shaft. The everting tube advances under fluid pressure in advance to the leading end of the primary catheter shaft.

The present invention, in one aspect, seeks to provide an improved liner.

Hitherto the liner, after removal from the patient, has simply been regarded as waste and has been disposed of.

The bacterial content of the colon is dynamic; not all microbial groups are present in each individual at all times. Medical treatment, geographical distribution, dietary and sanitary habits all affect the selection of resident microflora.

Infections of the bowel resulting in diarrhoea have long been studied, but the significance of the normal bacterial microflora in health and disease has received little attention. For example, there may be a role for altered bacterial microflora in conditions such as irritable bowel syndrome or chronic constipation.

It has been estimated that over 400 different species of bacteria comprise the normal intestinal flora of any given person. Although some details of this microflora can be found in microbiology texts, the Information available on normal colonic microflora is meagre. This data has mostly been obtained from examination of faecal specimens. The main disadvantage of this method being that the site within the colon of bacterial colonization for a particular microbe cannot be identified.

The present invention seeks, in one aspect, to provide improved data concerning the bacterial content of the colon, and other cellular content.

According to a first aspect of the present invention, there is provided a body cavity lining means as defined in claim 1.

The provision of two fluid chambers provides much greater control over the insertion and removal of the lining means. The provision of two separate fluid chambers allows the rigidity of the chambers to be controlled independently of eversion.

Of course, more than two fluid chambers could be provided, although this will result in a more complex structure.

Each of the chambers may include a flexible membrane. The flexible membrane could be latex or any other suitable flexible material. One of the chambers may also indude a relatively rigid member. For example, this relatively rigid member could be an endoscope, to which one of the flexible membranes is attached, the chamber being formed between the flexible membrane and the relatively rigid member.

The double walled portion of the lining means may extend for substantially half the length of the lining means, the remaining length of the lining means being single walled. This is convenient because, when the first chamber is everted to the maximum extent possible the double walled portion will extend from the inward tip of the first chamber to the outward end of the first chamber, which is external to the patient's body. The entire length of the lining means could be double walled, although this is not strictly necessary.

The chamber defined by the double walled portion may be divided into a plurality of packets.

Means may be provided for applying different fluid pressure to respective ones of the pockets. This will allow the rigidity of the liner to oe varied at different locations.

A pocket may be provided with valve means for allowing the passage of apparatus therethrough. This apparatus could be a surgical instrument.

Respective ones of the pockets may be positioned at longitudinally spaced intervals along the length of the lining means.

One or more of the fluid chambers may be pre-formed with a curved configuration. Alternatively, one or more of the chambers may be formed with a portion adapted to facilitate curvature of that portion, when required. These features may advantageously allow the lining means to readily conform to the configuration of a patient's colon.

The lining means may be provided with valve means mounted on one of the chambers, which valve means resists the passage of fluid from the lining means to the body cavity.

At least one of the chambers may be adapted to carry bacteria from the body cavity, which can be used for later analysis.

According to a second aspect of the present invention, there is provided a method of lining a body cavity, as defined in claim 20.

Because fluid can be separately applied to the fluid chambers, the rigidity of the chambers can be controlled separately.

A member may be inserted in the lining means, such that eversion of the first chamber causes the member to advance in the body cavity. As pressure is applied to the lining means to cause eversion, the first chamber will grip the member, due to application of pressure, and cause the member to advance with the chamber. This is a convenient way of advancing the member, which does not require the operator of the endoscope to push the member inwards.

Conveniently, the member is retracted by reducing fluid pressure to the first chamber whilst applying fluid pressure to the second chamber, the arrangement being such that the friction between the first chamber and the member is reduced whilst the fluid pressure in the second chamber causes the first chamber to remain in position in the body cavity. This is advantageous because the nature of eversion is such that the annular fold at the distal point of the everting chamber advances at half the speed of the inner surface of the chamber. This causes the member to advance within the body cavity at twice the rate that the distal point of the chamber advances. If the member was allowed to advance within the body cavity, during a colonoscopy, for example, the member could damage the colon because the protective chamber would not be present. The ability to retract the member without disturbing the chamber allows the person performing the colonoscopy to control the advance of the member by repositioning the member when the everting fluid pressure is not applied or is at a relatively low value.

The method as claimed in claim 20 may also include the step of withdrawing the lining means from the body cavity and analysing material deposited on the lining means.

As the liner is everted along the length of the body cavity, each part of the liner will contact a different part of the wall of the body cavity as the liner progresses. The liner is removed by re-everting and therefore capturing a "bacterial map" of the colon. The device may provide information not currently available about the colon. That is, not only can the type of bacteria be detected, but also the location of each type of bacteria within the colon can be determined.

The material may be analysed at a plurality of locations on the withdrawn liner, thereby providing an indication of the bacterial status at corresponding locations on the patient's colon.

The withdrawn liner may be separated into a plurality of parts and the material deposited on the separated parts may be analysed separately.

In the embodiment a first end of the liner is secured to a spindle and a second end of the liner is secured to a tubular member which is inserted into the opening of the body cavity. The liner between the first and second ends is colled around the spindle prior to eversion. Such an arrangement provides a convenient mechanism for storing the liner prior to eversion and also for storing the liner when it has been withdrawn from the body cavity and prior to analysis.

In the embodiment fluid applied to the liner causes the liner to uncoil from the spindle and evert into the body cavity.

The liner is conveniently withdrawn by rotating the spindle, so as to coil the liner about the spindle.

A semi-rigid or deformable tube or stent may be positioned between the first and second chambers. This may serve to provide a lumen within the body cavity even when no pressure is applied to the chambers and the endoscope may be removed.

Means for controlling the application of fluid by the first and second fluid applying means may be provided such that a member can be inserted into the body cavity as the first chamber everts. The controlled application of fluid may also enable a vacuum to be applied to one or both fluid chambers to facilitate removal.

Preferably a housing is provided for storing at least the first chamber before eversion and allowing release of the chamber during eversion. The housing may restrict the stored chamber material from exiting the housing until the chamber material is required as the member is advanced.

The housing may include an extendible cover portion for accommodating the chamber material as it is withdrawn from the body cavity. This extendible cover portion may collect the chamber material as it is removed from the body cavity, thereby allowing the chamber material to be discarded without coming into contact with other apparatus.

An enlargeable part for frictionally engaging the body cavity may be provided. The enlargeable part may be formed integrally with one of the chambers, providing an additional means for locating the chamber, or the enlargeable part may be formed separately from the chambers and have its own fluid supply. When a separate enlargeable part is provided, this may be used to distend the colon at the distal point of the first chamber to facilitate withdrawal of the first chamber from the colon.

One of the chambers may include a relatively rigid member, and means may be provided for advancing the rigid member within the body cavity so as to advance the first and second chambers within the body cavity. Means may be provided for advancing the rigid member by a first amount and subsequently withdrawing the member by a second amount, smaller than the first amount.

The method and apparatus of the invention could be used to train clinitians to perform a colonoscopy on cadavers or an excised or artificial body parts.

The term "body cavity" used In this specification should be interpreted to include a cavity of the human or an animal body but also includes cavities of inanimate bodies such as pipes.

For a better understanding of the present invention, an embodiment will now be described by way of example, with reference to the accompanying drawings, in which:-
Figures 1A and B show in cross section a conventional liner, such as described in US patent No. 5236423, before eversion and during eversion, respectively;
Figures 2A and B show in cross section a liner according to a first embodiment of the present invention before eversion and during aversion, respectively;
Figure 3 shows a cross-section through a liner in accordance with a first embodiment of the present invention;
Figure 4 is a cross-sectional view of the liner of the first embodiment being inserted into the body cavity of a patient;
Figure 5 is a cross-section taken along line A-A of Figure 4;
Figure 6 is a cross-sectional view showing the liner of the first embodiment fully inserted into the body cavity of a patient;
Figure 7 is a cross-sectional view showing apparatus for deploying the liner of the first embodiment;
Figures 8 and 9 show an alternative application of the liner of the first embodiment;
Figure 10 shows a cross-sectional view showing apparatus for deploying a liner according to a second embodiment of the invention;
Figures 11A to C show a modification to the second embodiment employing an additional resiliently deformable tubular liner;
Figures 12A and B show a modification to the arrangement illustrated in Figure 8;
Figure 13 shows a third embodiment of the invention comprising an inflatable balloon provided at the distal end of the liner;
Figures 14A and B show an expandable/collapsible stent for use with a fourth embodiment of the invention;
Figures 15A to C show the deployment of the stent in accordance with the fourth embodiment;
Figures 16A and B show a liner with a pre-formed curvature in accordance with a fifth embodiment of the invention;
Figures 17A and B show a modification to the fifth embodiment;
Figures 18A to C show a sixth embodiment of the invention including a valve formed on the liner;
Figure 19 shows the valve of the sixth embodiment at a different position, when the liner is at a different stage of eversion;
Figure 20 shows a partly cross-sectional exploded view of the apparatus for performing body cavity analysis;
Figure 21 shows a cross-section taken along the line A-A of Figure 20;
Figure 22 shows a cross-section through a liner;
Figure 23 is a cross-sectional view showing a liner fully inserted into the body cavity of a patient;
Figure 24 shows the liner stored after withdrawal from a patient's body cavity;
Figure 25 shows schematically the cutting of the body cavity liner into a series of sections for analysis;
Figures 26A and 26B show the correction of looping of a colon according to a further embodiment of the invention;
Figure 27 shows a cross-sectional view of a liner including a rigid support within the liner according to another embodiment of the invention; and
Figures 28A and 28B show the provision of fluid pockets in a membrane according to an additional embodiment of the invention; and
Figures 29A and 29B show access valves provided at the distal end of a membrane according to another embodiment of the invention.

In the drawings like elements are generally designated with the same reference numeral.

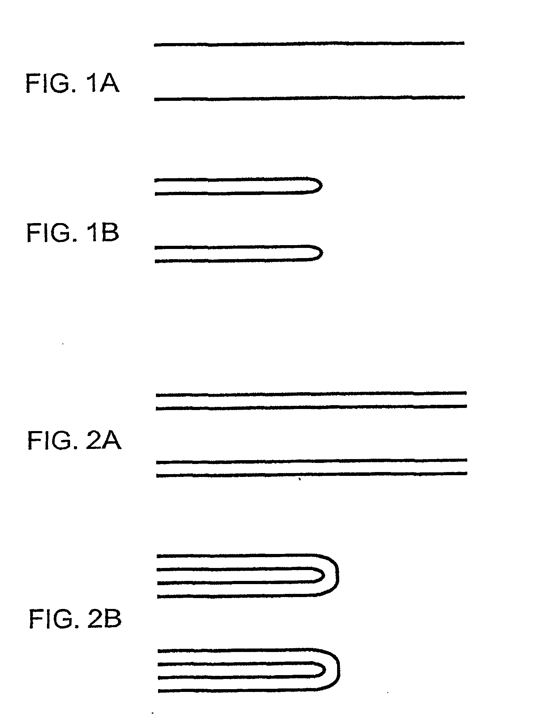

Figures 1 and 2 illustrate the principle of the first embodiment of the invention with reference to the prior art. As shown in Figure 1, in the prior art a single walled liner is formed (as shown in Figure 1A). As the liner everts into the patient's colon the single walled liner is folded back over itself (as shown in Figure 1B). In contrast, in the first embodiment of the present invention, the liner is formed with a double walled portion, as shown in Figure 2A. When the double walled liner is everted into the colon the liner folds back over itself, forming, in section, four concentric walls extending longitudinally within the colon.

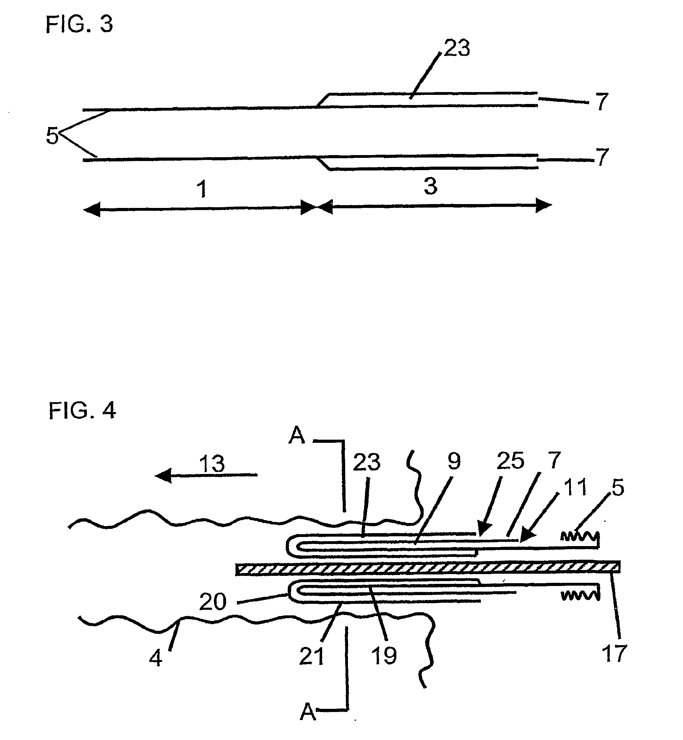

Figure 3 shows a liner of the first embodiment in its manufactured form, before use. The liner may comprise polyurethane or latex, or any other suitable eversible material known to those skilled in the art. The liner includes a first portion 1 which is single walled and a second portion 3 which is double walled. Each of the portions 1 and 3 is 1.8 metres in length, which is generally the maximum distance that an endoscope is inserted into the human colon to perform a colonoscopy. Of course, the length of portions 1 and 3 will be varied depending on the particular application. For example, if the patient is an animal, the lengths of the portions 1 and 3 will be adjusted in accordance with the length of the animal's colon. It is preferable that portions 1 and 3 are of substantially equal length.

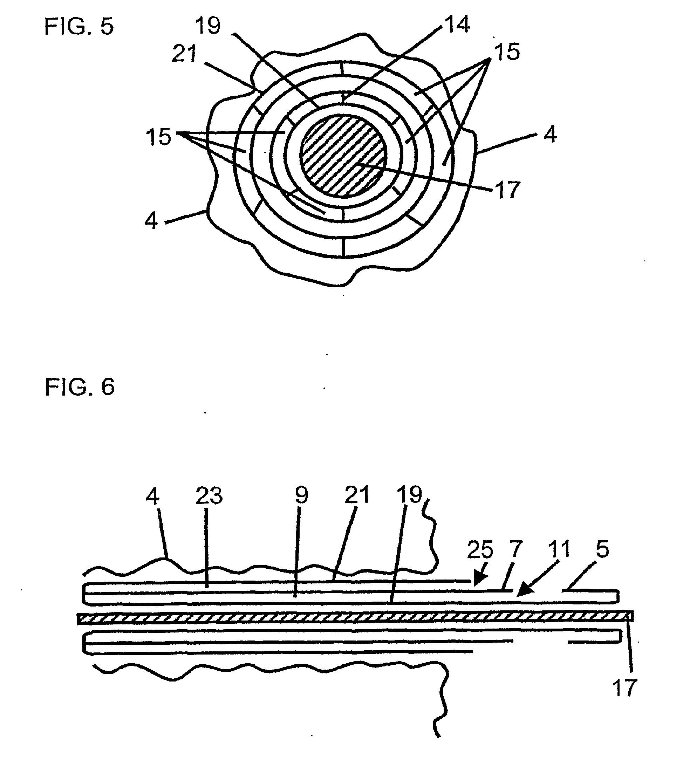

Figures 4 and 6 show how the liner is inserted into the colon 4. A first end 5 of the single walled portion 1 of the liner is fixed in position on an inserting device not shown in these figures. A second end of the double walled portion 3 of the liner is fixed at position 7 on the device. Initially, the second end of the liner at position 7 is folded over itself to form a first chamber 9.

Fluid enters the first chamber 9 at point 11. The fluid causes the liner to evert and travel up the colon 4 in the direction of arrow 13 until the liner is everted sufficiently to line the required length of the colon 4, as shown in Figure 6.

Figure 5 shows a cross-section taken along line A-A of Figure 4. This cross-section shows how the double walled portion 3 of the liner is structured. At six equally spaced locations around the circumference of the double walled portion 3 of the liner the respective walls are joined by webbing 14 (or by welds, not shown) to form a plurality of fluid pockets 15. There could, of course, be more or fewer than six joins between the walls, and these need not be equally spaced.

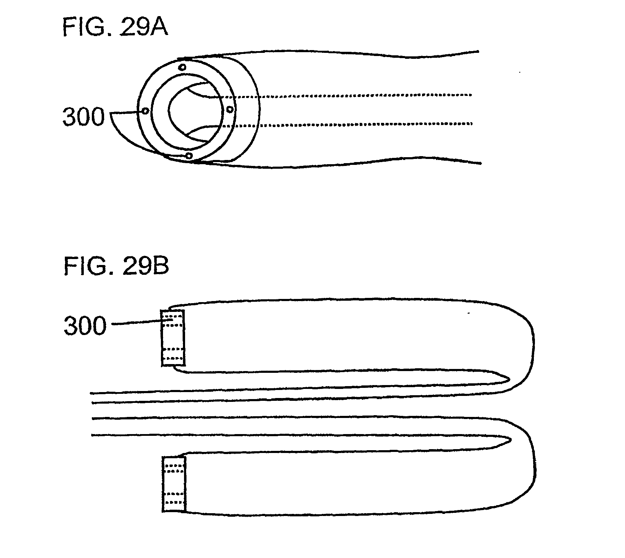

When the liner is in the desired position within the colon 4, one or more of the pockets 15 can be used as a channel down which a surgical instrument, or a semi-rigid member, may be passed. When the surgical instrument reaches the distal end of the liner, it will need to puncture the distal end to emerge from the liner so that the instrument can have access to the colon. The pockets 15 which are not acting as channels for surgical instruments may be configured so that they can have a supply of fluid pressure maintained thereto, if required. Alternatively, rather than the surgical instrument or other semi-rigid member puncturing the distal end of the liner, the liner may be provided with one or more access valves at the distal end thereof (in use), as shown in Figure 29. With this arrangement, instead of there being a requirement to puncture the liner, the access valve or valves 300 will allow the surgical instrument or semi-rigid member to pass therethrough. Preferably, the access valve or valves 300 are configured to form a fluid-tight seal around the surgical instrument/semi-rigid member passing therethrough, to maintain any desired fluid pressure in the pocket in which the surgical instrument/semi-rigid member is positioned. Of course, the access valve or valves 300 may be provided in a liner which is not divided into a series of pockets.

As the liner is initially inserted in the colon an endoscope 17 is positioned within the liner. Pressure applied at point 11 to the chamber 9, which causes the liner to evert, will also push the liner against the endoscope 17 so that the endoscope 17 is gripped by the liner and carried by the liner during eversion.

The nature of the eversion process is such that the inner layer 19 of the liner, which presses against the endoscope 17 will progress at twice the rate of the distal point 20 of the liner. Hitherto, it has been difficult for the operator to control the advancement of the endoscope 17 by grasping the end of the endoscope 17 that is external to the body cavity, because the pressure caused by the eversion process locks the inner layer 19 of the liner against the endoscope 17. If eversion pressure was reduced to allow the endoscope 17 to be repositioned, this was likely to cause the position of the liner within the colon 4 to be disturbed, and could even cause undesired folds to be formed in the liner.

To overcome this problem the liner in accordance with the first embodiment of the present embodiment includes the double walled portion 3 which forms a second fluid chamber 23. Fluid is applied to second fluid chamber 23 at point 25. The supply of fluids at points 11 and 25 is independently controllable so that the pressure in first chamber 9 and second chamber 23 can be controlled separately. Adjacent pockets 15 formed in the double walled portion 3 of the liner are in fluid communication and allow fluid applied at point 25 to pass from pocket to pocket.

During eversion of the liner a constant pressure may be applied to the second chamber 23. The pressure could be varied, for example, to compensate for different extant emplacement lengths, or if muscular contraction occurs during the insertion process.

When eversion begins, and the endoscope 17 is advanced by contact with the inner layer 19 of the liner, so that the endoscope 17 begins to extend into the colon 4 beyond the distal end 20 of the everting liner. Pressure within the first chamber 9 is then reduced by controlling the fluid pressure applied at point 11. This enables the operator or advancing apparatus to grasp the external end of the endoscope 17 and reposition the endoscope so that it does not extend beyond the liner, the reduction in pressure in chamber 9 reducing the grip between the liner and the endoscope 17. However, the generally constant pressure to the second chamber 23 is maintained, which prevents the position of the liner within the colon 4 from being disturbed and also prevents undesired folding of the liner. When repositioning of the endoscope 17 is completed, fluid pressure to chamber 9 is increased and the liner is further everted. The reduction in pressure to the chamber 9 is repeated periodically to allow for adjustment of the position of the endoscope 17 by the operator until the endoscope 17 is fully inserted in the colon 4 and the liner is everted sufficiently to line the required length of the body cavity, as shown in Figure 4. When the liner is at its maximum length, the inner layer 19 of the liner will comprise the single walled portion 1 of the liner and the outer layer 21 will comprise the double walled layer 3 of the liner. Of course, in many applications the liner will never reach its maximum length.

By controlling the advance of the endoscope 17 so that its inward tip is maintained generally in line with the distal point 20 (inward extent) of the liner, the operator has constant vision, provided by the endoscope, during insertion of the endoscope. Hitherto, in methods where a liner was everted prior to insertion of the endoscope, the walls of the colon could generally only be properly inspected as the endoscope (and liner) were withdrawn from the colon.

When the endoscope 17 is fully inserted (Figure 4) reduction in pressure to the first chamber 9 of the liner and maintenance of constant pressure to the second chamber 23 of the liner will allow for the removal of the endoscope 17 without disturbing the position of the liner within the colon 4. An alternative instrument to the endoscope 17 may then be inserted into the colon 4 through the liner.

To remove the liner from the colon 4, pressure applied to second chamber 23 is reduced while pressure is maintained in first chamber 9. As a result, friction occurs between the endoscope 17 and the inner layer 19 of the liner during retraction of the endoscope 17 thus causing the liner to invert The liner progresses backwards with the endoscope 17. When the distal tip of the endoscope 17 emerges from the liner half the previously everted part of the liner will have been inverted, the inner layer 19 of the liner travelling at twice the rate of the distal point 20 Pressure to the first chamber 9 is reduced and the endoscope 17 is reinserted through the liner. When the tip of the endoscope 17 reaches alignment with the inward end of the liner, pressure is reapplied to first chamber 9 and the endoscope 17 is again retracted. Alternatively, removal of the liner can be achieved by maintaining an amount of pressure in chamber 23 and physically withdrawing the liner from point 11. It is also possible to exert a vacuum at points 25 and 11 therefore causing the liner to collapse against the endoscopes 17 to facilitate removal.

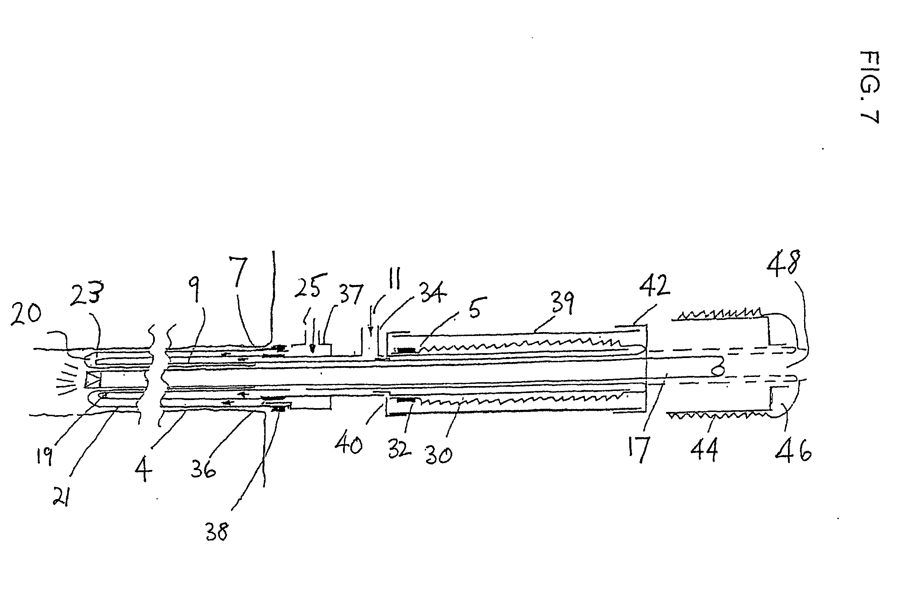

Figure 7 shows the device used for inserting the liner and the endoscope 17. The device comprises a storage member 30 in the form of an open-ended cylinder. The first end 5 of the liner is fluid-tightedly sealed to the storage member 30 by seal 32. The storage member 30 stores the liner by having the liner gathered over the storage member 30 in concertinered form. The liner passes along the outer surface of the storage member 30 and then back on itself along the inner surface of the storage member 30 towards the colon 4, in use. A first wall of the second end 7 of the liner is connected to a first fluid source 34 and is sealed thereto by seal 36. The other wall of the end 7 of the liner is attached to second fluid source 37 by seal 38. Fluid sources 34 and 37 are operable to apply fluid to first and second fluid chambers 9 and 23, respectively.

Although not shown in Figure 5, a rigid tube may be mounted around the front end of the device to facilitate transanal insertion of the device beyond the patient's sphincter. The liner everts from this tube.

A liner-retaining cylinder 39 surrounds the storage member 30. The retaining cylinder 39, together with front end cap 40 and rear end cap 42, serve to restrict the liner from leaving the storage member 30 until this is required in the eversion process.

The endoscope 17 passes through a resulting passage in the retaining cylinder 39, end caps 40 and 42 and fluid sources 34 and 37 The operator can grasp the end of the endoscope 17 which emerges from rear end cap 42 in order to control insertion or retraction of the endoscope 17 within the patient's colon 4.

When it is desired to withdraw the liner from the patient's colon the rear end cap 42 is removed, which allows the liner to progress rearwards with the endoscope 17 as this is also withdrawn. When the end cap 42 is removed a disposable cover 44 is fitted over the retention cylinder 38. The cover 44 has a generally closed end 46, although this closed end does include a channel 48 which has a diameter slightly larger than the diameter of the endoscope 17 in order to allow the passage of the endoscope 17 therethrough. As the endoscope 17 is withdrawn through the channel 48 the liner is collected within the cover 44, the channel 48 being sized so as to prevent the passage of the liner therethrough as the endoscope 17 is retracted. The presence of the cover 44 allows the withdrawn liner to be disposed of, after detaching seals 32, 36 and 38, without the liner needing to make contact with other apparatus and possibly causing contamination.

The cover 44 is made of a material which can expand axially in order to accommodate the liner as it is withdrawn from the colon.

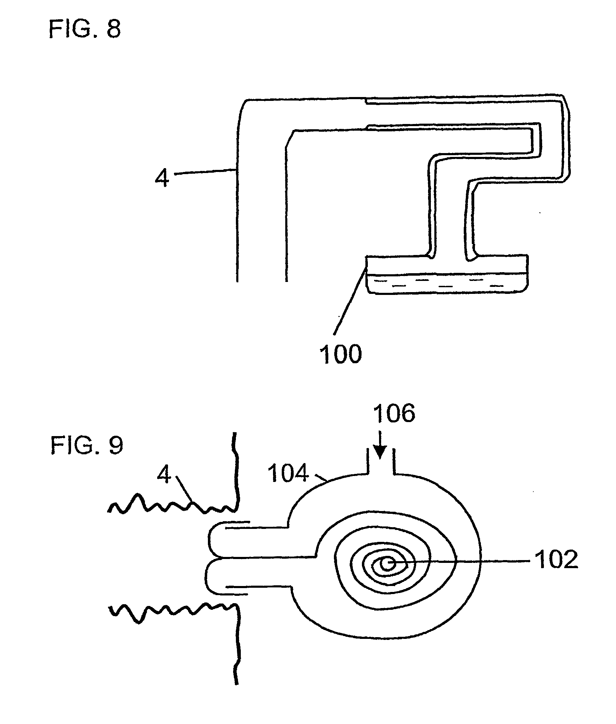

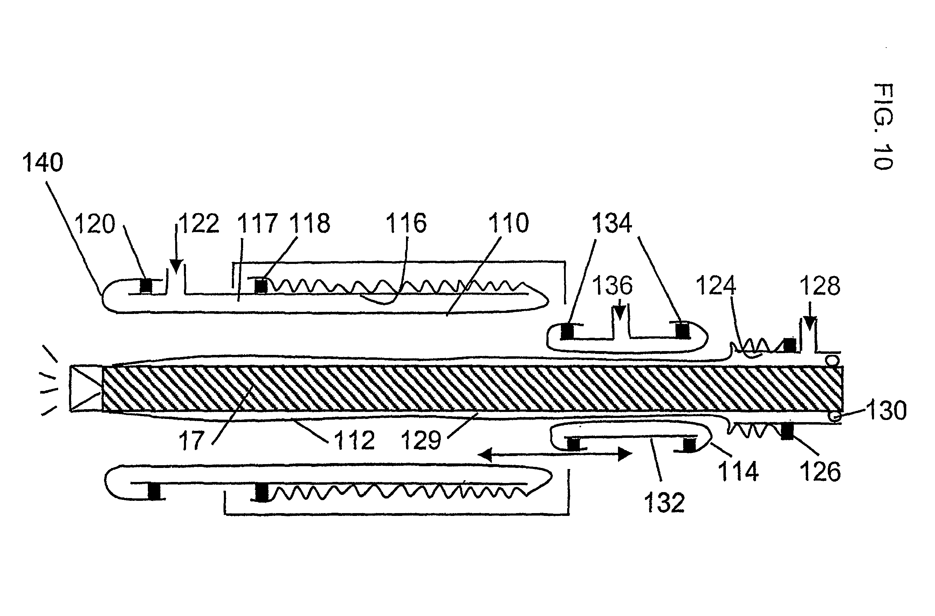

Figures 8 and 9 show an alternative use of the body cavity liner. Here the liner is everted approximately half way round the colon 4 (Figure 8). The external end of the liner is connected to a colostomy bag 100. The installed liner allows liquid waste from the patient to be collected in the colostomy bag. Because in this embodiment no device, such as an endoscope, needs be to be inserted within the liner, the liner can be stored by winding it around a central spindle 102 inside a cylindrical casing 104. The liner is everted by applying fluid at a port 106.

The liner can be similarly applied to line a patient's urinary tract. In this application the required length of the liner is relatively short and it is not necessary to coil the liner.

Although not shown, the liner has a double walled structure as shown in Figures 3 to 7. In both the applications the liner is maintained in position and a clear passage for waste is formed by applying fluid pressure to the double walled portion 3 of the liner.

A particular advantage of the first embodiment is that the liner can be everted along the colon using a low fluid pressure (0.2 to 0.5 bar) without the presence of an endoscope (or similar instrument). The prior art single-walled liners cannot do this because the eversion pressure would be too high and may damage the colon.

To facilitate eversion of the liner without an endoscope being present, a reciprocable member can be positioned within the storage member 30 and the liner, which is slightly smaller than the internal diameter of the storage member 30. Advancement of the reciprocable member in the direction of the distal end of the colon 4 will push fluid towards the distal end of the liner, thereby freeing up stored liner material and advancing the liner within the colon 4. Repeated reciprocation of the reciprocable member will provide continued advancement. The reciprocable member may be a solid, cylindrical rod, or it may be an inflatable member with repeated inflation and deflation providing or mimicking the reciprocation. This arrangement allows eversion of the liner at low pressure.

Figure 10 shows an example not forming part of the claimed invention which employs two separate liners or membranes 110 and 112. A further inflatable membrane 114 is also provided to advance the endoscope within the membrane 110.

The first membrane 110 is mounted on a storage member 116 in the form of an open-ended cylinder, in a similar manner to the first embodiment. At one end the first membrane 110 is fluld-tightedly seated to the storage member 116 by seal 118. The other end of the first membrane 110 is fluid-tightedly sealed by seal 120 to the distal end of the cylindrical surface which forms the storage member 116. A fluid source 122 is operable to apply fluid pressure to the chamber 117 of the first membrane 110 between its sealed ends.

The second membrane 112 is sealed by means (not shown) to the distal end of the endoscope 17. Material of the second membrane 112 is stored in concertinered form around a second storage member 124, and is fluid-tightedly sealed to the second cylindrical storage member 124 by seal 126. An O-ring seal 130 provides a fluid-tight seal between the storage member 124 and the surface of the endoscope 17. As the endoscope 17 is inserted or removed from a patient the endoscope 17 will slide with respect to the storage members 116 and 124. The O-ring seal 130. is operable to maintain a fluid tight seal despite this relative movement. A second fluid source 128 is operable to apply fluid pressure to the chamber 129 formed between the surface of the endoscope 17 and the membrane 112.

The third membrane 114 is mounted around the internal surface of a third storage member 132, which has the form of an open ended cylinder. The ends of the third membrane 114 are sealed to opposite ends of the outer surface of the storage member 132 by respective seals 134. A third fluid source 136 is operable to apply fluid to the third membrane 114 between its sealed ends.

The first and second storage members 116 and 124 are mounted in fixed relationship to one another, whilst the endoscope 17 is mounted for longitudinal movement within these members 116 and 124.

To advance the endoscope 17, pressure is applied to first fluid source 122, which will cause the membrane 110 to expand, gripping the endoscope 17 and the membrane 112 mounted around the endoscope (the membrane 112 not having fluid pressure applied thereto at this time). Fluid pressure is then applied to the third membrane 114 using the third fluid source 136. The inflated membrane 114 will grip the endoscope 17 and the second membrane 112. The storage member 132, together with the membrane 114 attached thereto, is then moved forward in direction A by approximately 50mm or greater. The action of moving the storage member 132 and membrane 114 in the direction A by approximately 50mm or greater will also advance the endoscope 17 and the membrane 112 wrapped around the endoscope by 50mm by virtue of the pressure applied by the membrane 114. The advancement of the endoscope 17 will draw stored material of the membrane 112 from the second storage member 124. As explained earlier, the annular fold at the distal end 140 of the liner 110 will advance at half the rate of travel of the liner 110 along the inner surface of the storage member 116. This will result in the endoscope 17 advancing at twice the rate of the liner 110.

Next, fluid pressure is released from fluid source 122, allowing the membrane 110 to deflate. Whilst pressure to fluid source 112 is released, fluid pressure is momentarily applied to the fluid source 128, the membrane 112 thereby being briefly inflated then deflated. This action will force the membrane 110 away from the endoscope 17, thereby releasing frictional resistance.

The third storage member 132, with the third membrane 114 still inflated is then moved approximately 25mm in direction B, bringing the viewing portion 138 at the distal end of the endoscope 17 adjacent to the distal end 140 of the first membrane 110.

The pressure to the third membrane 114 is then released, thereby deflating the membrane 114, and removing the gripping force from the endoscope 17. The third storage member 132 is then moved a further 25mm (approximately) in direction B, so that it is in the position it occupied before movement in direction A.

With each motion of the storage member 132 in direction A, the first membrane 110 is advanced by 50mm. The subsequent retraction by 25mm of the endoscope 17 does not retract the first membrane 110. The example allows the endoscope to be progressively and controllably advanced within the colon with the first membrane 110, so that the viewing portion 138 of the endoscope 17 is always within 25mm of the distal end 140 of the first membrane 110, thereby maintaining a good view of the surface of the colon during eversion of the first membrane 110. The continued reciprocation of the third storage member in direction A and then in direction B may be provided by an automated mechanical linkage which could, for example, be under the control of a computer. This is highly advantageous compared with prior art systems requiring manual manipulation of the endoscope 17 within the everting membrane. Also, the pressure required to advance the present device is between 0.2 and 0.5 bar, much lower than the prior art.

It should be understood that in prior art methods, high pressure within the liner/membrane pushes the endoscope around the colon. The high pressure makes the liner fairly rigid - which may be painful for the patient and probably dangerous if the liner was to burst. In contrast, in the second embodiment, the membrane 110 is inflated to a lower pressure so that the liner just grips the endoscope. The pressure is not required to be high enough to carry the endoscope forward. However, the inflated liner does gently open up the bowel and thereby reduces the pushing force required to advance the endoscope 17. The pushing force is applied by the third membrane 114 to the endoscope 17. The endoscope 17 leads the membrane up the colon, rather than the membrane pushing the endoscope 17, as in conventional methods. By using the endoscope 17 controls, the endoscope can be directed around curves in the colon. The endoscope will guide the membrane around the curves.

It should be appreciated that, in any of the embodiments, the membrane or liner does not have to pass up the full length of the colon. The endoscope 17 may extend, for example, twice the distance of the membrane or liner.

For example, the membrane may evert with the liner for half the length of the colon. Thereafter, the endoscope may be allowed to proceed at its natural speed, which is twice the speed at which the membrane everts. Sometimes it is the curvature of the colon nearest the anus which is most difficult for the endoscope to negotiate - and the membrane will help with this. The inner parts of the colon may be easier to negotiate, so that the membrane is no longer required to advance with the endoscope. In the second embodiment, the travel of the third membrane 114 in direction A could be more than 50 mm, for example 250 mm. The travel of the third membrane 114 in direction B could be reduced or minimised. These movements could be varied as the endoscope travels up the colon, according to the conditions.

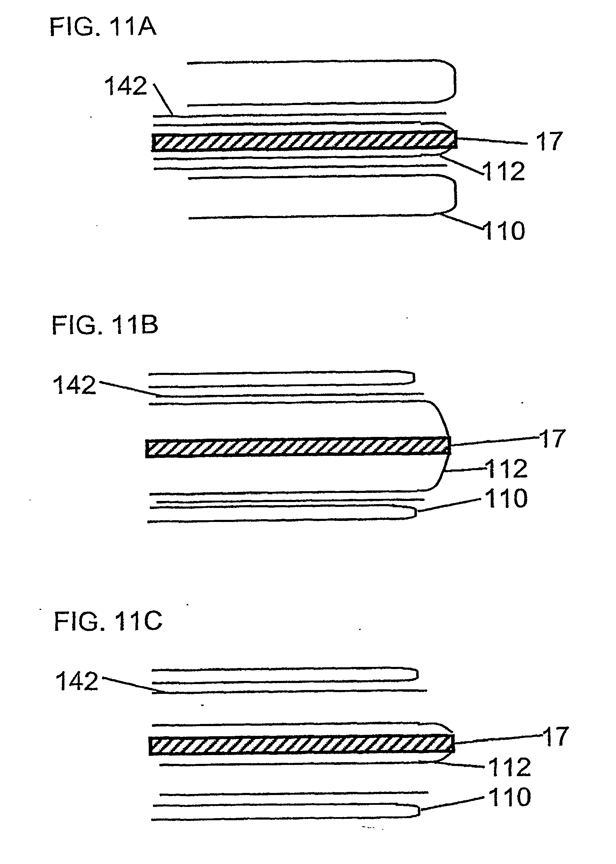

Figure 11 shows a modification to the example. In this example a resiliently deformable tubular liner 142 is provided over the endoscope 17 and the second membrane 112, and within the first membrane 110. The tube 142 may be inserted over the endoscope and first membrane 110 after full insertion of the endoscope 17 within the colon, whereafter the endoscope 17 may be withdrawn (together with the second membrane 112). This leaves a resilient lining 142 within the colon which allows the insertion of other tools, for example those to perform endoluminal surgery.

Thus, a central lumen is provided without requiring any inflation of the first membrane 110.

Figure 11A shows the arrangement of the tube 142 when the first membrane 110 is inflated and the second membrane 112 is deflated. In this configuration the tube 142 is compressed to reduce its circumference. In Figure 11B the first membrane 110 is deflated and the second membrane 112 is inflated. In Figure 11B the tube 142 has retumed to its normal circumference by virtue of its resilience and also by virtue of pressure applied by the second membrane 112. In Figure 11C both the first membrane 110 and the second membrane 112 are deflated. This leaves a cavity 144 around the endoscope 17. It is a now a simple matter to withdraw the endoscope, leaving an uninterrupted cylindrical cavity within the colon which is lined by the tube 142. Surgical Instruments could be passed up this cavity to perform surgery on the colon. Alternatively, the endoscope 17 could remain in place, with the annular cavity 144 formed there around providing access for surgical instruments.

In this example the tube 142 is of circular cross section when at its non-compressed, rest position.

It should also be appreciated that the tube 142 could be inserted simultaneously with the endoscope 17. Because the tube 142 is resiliently compressible, it will not interfere with the actions of the first, second and third membranes, 110, 112, 114, during deployment of the endoscope 17/tube 142. A resiliently deformable tube 142 may also be employed with the double walled liner of the first embodiment.

If the endoscope 17 is deployed in accordance with the second embodiment of the invention, even without the resiliently deformable tube 142, by applying fluid pressure from fluid source 122 it is possible to remove the endoscope 17 and second membrane 112 whilst maintaining the first membrane 110 in position within the colon, leaving the colon lined by the first membrane 110. It will be possible to subsequently reinsert the endoscope 17. A reduction in fluid pressure from fluid source 122 may assist in the reinsertion of the endoscope 17. With such an arrangement, to facilitate removal and/or reinsertion of the endoscope 17 a lubricant may be applied to one of the first and second membranes 110, 112 to reduce friction between the respective membranes.

Having the first membrane 110 in place in the colon and inflated by application of fluid pressure from fluid source 122 the colon can be made stiffer. This can be advantageous in laparoscopic surgery. The stiffness makes the bowl easier to handle, and may reduce the number of laperoscopic instruments required to hold the bowel during the operation, i.e. the membrane 110 acts as an endoscopic retractor during laperoscopic surgery. The apparatus of the first embodiment is also suitable for providing rigidity to the colon when the endoscope is not present.

By applying a negative fluid pressure to the fluid source 128 the second membrane 112 will be sucked onto the surface of the endoscope 17. This may assist in removal of the endoscope 17 from the colon. Further, the first membrane 110 may be sucked, by application of a negative pressure, onto the second membrane 112 to ease removal of the endoscope 17 and the first membrane 110.

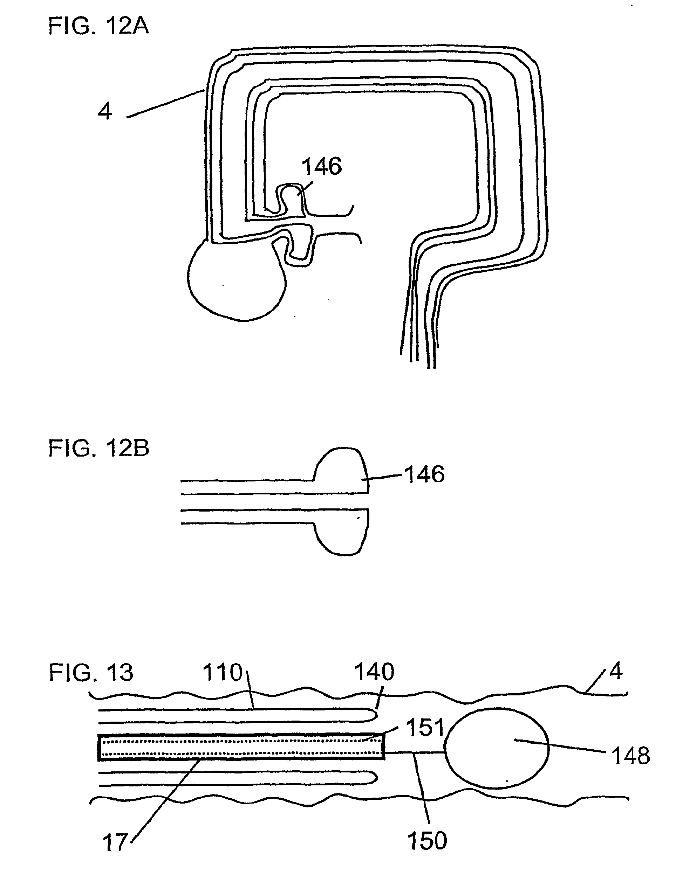

Figure 12 shows a modification to the arrangement illustrated in Figure 8. In the Figure 12 arrangement the double walled liner has an inflatable/expandable portion 146 at its distal end. When the liner is deployed to the appropriate extent within the colon the inflatable/expandable portion 146 is inflated. The inflation of the portion 146 serves to anchor the liner in position in the colon. For example, the liner may be passed through the ileocaecal valve, whereafter the portion 146 is inflated. This allows the bowel contents to be collected in the lumen of the liner, where free passage can be controlled by inflation and deflation of pockets 15 of the double walled liner. A lumen can also be provided by providing a resilient tube 142 like the tube 142 of Figure 11. A bag similar to the bag 100 illustrated in Figure 8 may be provided at the proximal end of the liner to store the bowel contents. This is essentially a faecal catheter.

In a third embodiment of the invention (Figure 13) an inflatable balloon 148 is provided spaced apart from the distal end 140 of the membrane 110. Two fluid channels 150 and 151 pass through a channel (not shown) formed in the endoscope 17. A fluid supply (not shown) is provided externally of the colon for inflating the balloon 148, when desired. When It is required to remove the endoscope 17 from the membrane 110, the balloon 148 is inflated via the fluid channel 150. Fluid pressure is then applied via channel 151 to exert pressure between the balloon 148 and the end of the liner to assist the tension force required to remove the liner by pulling the endoscope from outside the colon. The balloon 148 may be stored in a deflated state within the endoscope 17 when not in use, or the balloon may be kept separately from the endoscope and passed through the channel in the endoscope to the distal end thereof when required.

Of course, inflation of the balloon 148 is also advantageous if it is desired to remove the membrane 110, or any other item in the colon 4, as well as the endoscope 17. As an alternative to deployment of a balloon 148, some other means for providing mechanical force or fluid pressure could be provided.

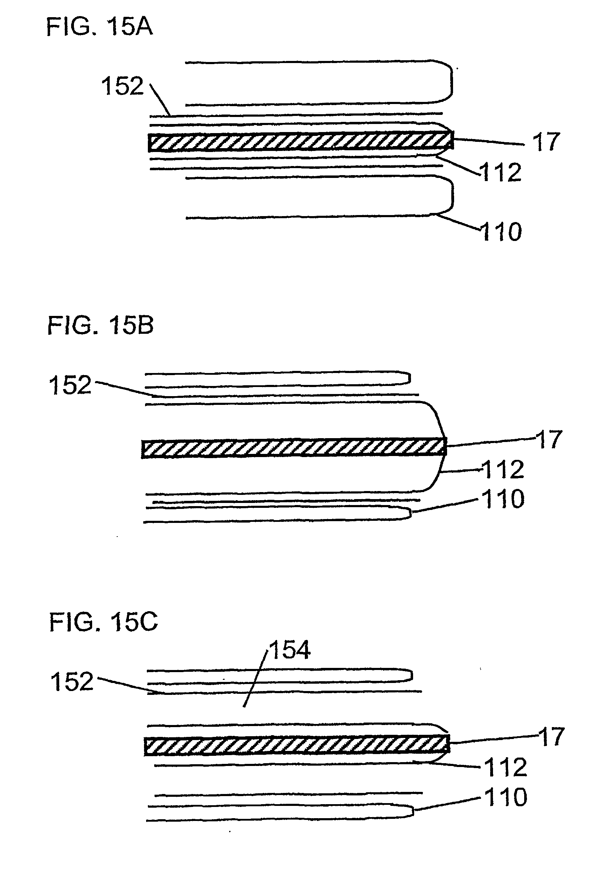

Figure 14 shows a fourth embodiment of the invention. In this embodiment a stent 152 is provided for use with the apparatus of the second embodiment of the invention. Medical stents 152 will be known to those skilled in the art. Essentially, they comprise a tubular structure which has a circumference which can be increased or decreased by application of pressure. The expandable/collapsible stent 152 is loaded onto the endoscope 17 over the second membrane 112. The endoscope is then deployed to the appropriate position within the colon 4, in the manner as described in relation to Figure 10. The desired location could, for example, be near to site of multiple polyps. At the selected location the membrane 112 is inflated, whilst the membrane 110 is deflated. The inflation of the membrane 112 will expand the stent from the position shown in Figure 14A to the configuration shown in Figure 14B. The stent 152 will push against the deflated membrane 110, and the stent 152 will, due to its inherent characteristics, remain in this configuration until an opposite force is provided. The membrane 112 is then deflated, and a hollow chamber is created between the walls of the endoscope 17 and the walls of the stent 152. Surgical instruments may then be passed down the colon 4 between the deflated membrane 110 (with the stent 152 at its distal end) and the deflated membrane 112. Alternatively, the endoscope 17 could be removed from the colon 4, allowing greater access for surgical instruments. Tissue specimens, for example, large polyps, can be removed either in the channel formed between the endoscope 17 and the membrane 110, or through the uninterrupted channel if the endoscope 17 is removed. When procedures in the area of the stent 152 are completed, the endoscope 17 is redeployed (if it has been removed). The membrane 110 is then inflated, which causes the stent 152 to collapse to the configuration shown In Figure 14A. In its collapsed state the stent 152 is pressed against the deflate membrane 112 and endoscope 17. The collapsed stent 152 can then be removed from the colon 4 by withdrawal of the endoscope 17. Of course, it will be understood by those skilled in the art that the arrangement will also be effective in other body cavities such as the urinary tract, the vascular system, etc.

Figure 15 shows the arrangements of the membranes 110, 112 and the stent 152 at various stages of deployment. Figure 15A shows the stent 152 in a collapsed state around the endoscope 17 and the membrane 112. The Figure 15B shows the membrane 112 in an inflated state which expands the stent 152 within the membrane 110. Figure 15C shows the membrane 112 deflated, leaving an annular cavity 154 between the inner surface of the stent 152 and the outer surface of the endoscope 17 further example membrane 112.

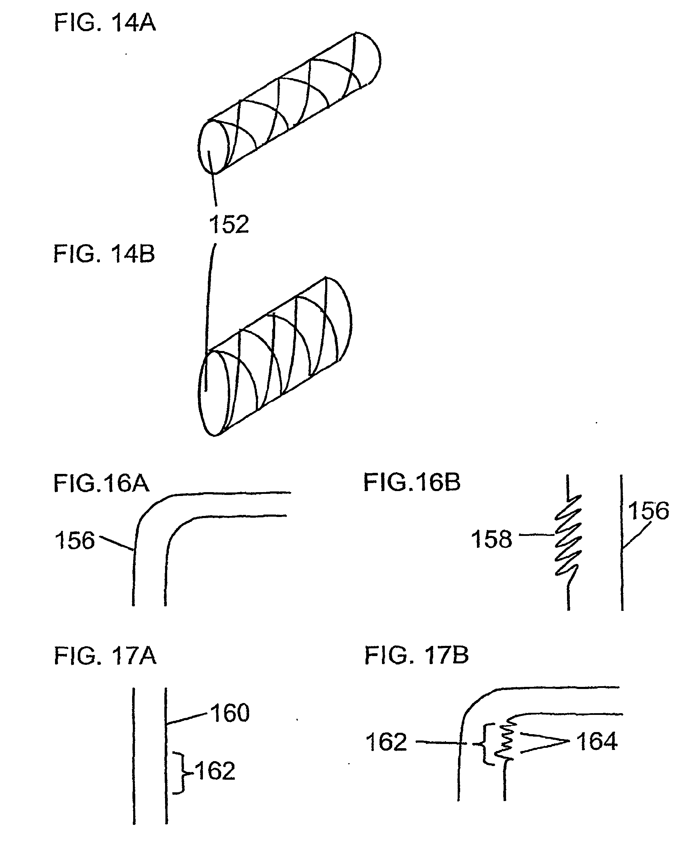

Figure 16 shows a further example. In this example a membrane or liner 156 is formed so as to have a curvature in its natural state, as shown in Figure 16A. As shown in Figure 16B, the liner 156 can be arranged so that it has a linear form, which is convenient for storage and also during deployment of the liner 156 within the colon 4. The liner 156 is arranged so that, when it its linear form, a series of pleats or folds 158 form in the surface which would normally be the outside of the pre-formed curved. When no force is applied to the liner 156, it will return to its curved state as illustrated in Figure 16A. Such an arrangement is advantageous because the curvature will allow the liner 156 to take the natural shape of the colon 4 when it is deployed. The pre-formed curves may help direct the membrane through, for example, 90° curves of the colon. This may allow the pressure required to deploy the membrane to be reduced, which is particularly advantageous if a single walled liner is being used. The arrangement may facilitate the insertion of a membrane without simultaneous insertion of an endoscope or other semi-rigid member.

Figure 17 shows a modification to the Figure 16 arrangement. The liner 160 has a region 162 shown in Figure 17A which is manufactured so that it will readily form a series of folds to facilitate curving of the liner 160 when deployed in a curved body cavity such as the colon 4. Figure 17B shows the liner 160 in a curved state. A series of pleats 164 can be seen to have been formed in the region 162 where the curvature of the liner 160 is at its greatest. The liner has different rigidity at different points along its length - so that some parts will tend to remain straight and some will tend to form curves corresponding to the shape of the colon. Essentially these pleats can be preformed or formed during the operating procedure by welding or fixing by some other means to direct the inside liner to conform to the shape of the colon.

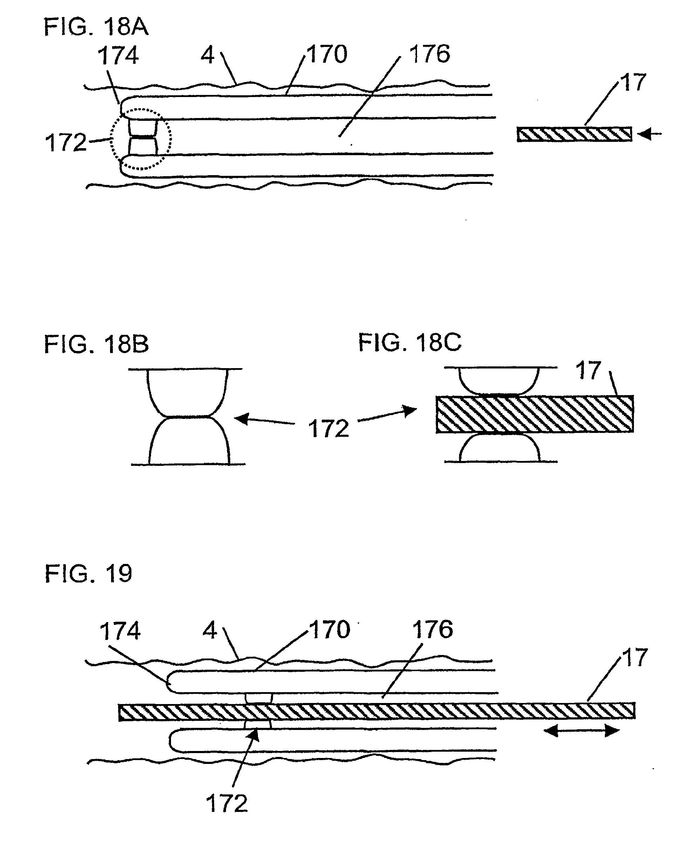

Figure 18 shows a further example. In Figure 18 a single walled liner 170 is shown, although this liner could have a double walled structure like the double walled structure of the liner of the first embodiment. The liner 170 has a valve 172 positioned halfway along the length of the liner (before deployment). In order to deploy liner 170 within the colon 4, the valve 172 will be in a closed position to maintain fluid pressure for eversion. When eversion is completed the valve 172 will be positioned coincident with the distal end 174 of the liner 170 within the colon 4. The valve 172 may be of a similar configuration to the heart valve of a person, and will operate in a similar manner.

When the liner 170 is fully everted within the colon 4, as shown in Figure 18A, the valve 172 at the distal end of the liner 170 allows the formation of a chamber 176. This chamber 176 may be inflated by application of fluid pressure to the chamber 176, pushing the liner 170 against the colon 4. The valve 172 may be fluid-tight, or will at least resist the passage of fluid flow therethrough (Fig. 18B). Once the liner is deployed, as the endoscope 17 travels up the chamber 176 the valve 172 will be operable to allow the release of excess fluid pressure which is trapped between the distal end of the endoscope 17 and the valve 172. When the endoscope 17 reaches the valve 172, the valve is operable to open to the extent to allow the endoscope 17 to pass therethrough, as shown in Figure 18C. Parts of the valve may form a seal around the endoscope 17, maintaining a fluid tight or fluid resistant barrier. Figure 19 shows the valve 17 spaced apart from the distal end 174 of the liner 170. The valve 17 may occupy this position when the liner 170 is not everted to its maximum length. The valve 172 will also occupy this position momentarily during eversion and invertion of the liner 170 within the colon 4. The endoscope 17 is shown passing through the liner 172. The liner 170 may be deployed and everted prior to insertion of the endoscope 17, or the endoscope 17 may be advanced as the liner 170 is everted. Further the valve 172 can be operated at any stage during inversion to facilitate movement of the endoscope 17.

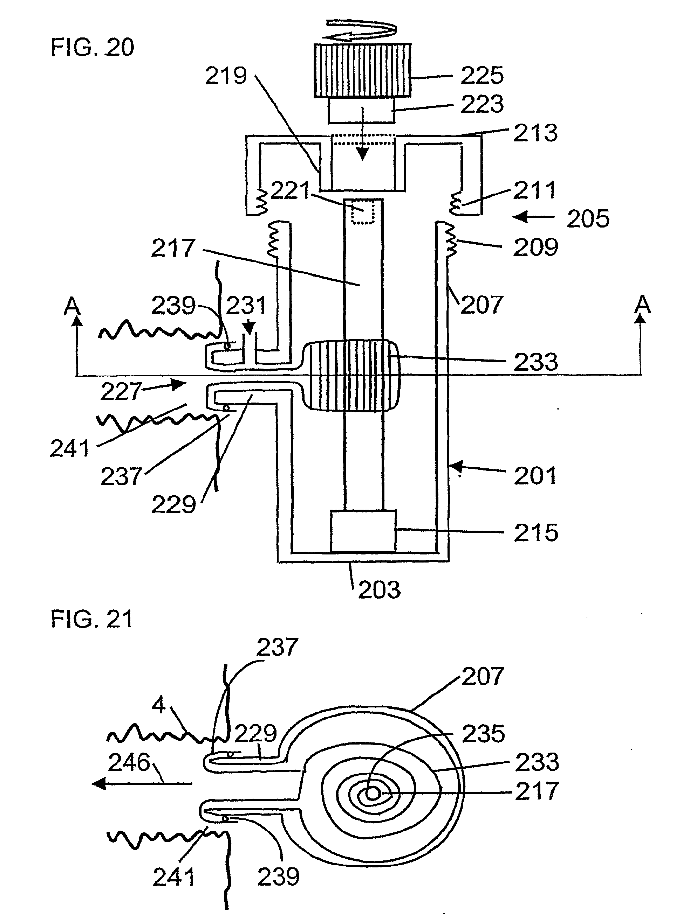

A further embodiment will now be described for providing bacterial data from the colon. The apparatus illustrated in Figure 20 comprises a generally tubular main body 201 which is closed at one end 203. At the open end 205 of the main body 201 the side walls 207 include an externally threaded portion 209. The externally threaded portion 209 co-operates with the internally threaded portion 211 of an end cap 213 which serves to close the main body 201.

The internal surface of the closed end 203 of the main body 201 has a hollow cylindrical part 215 extending therefrom. The cylindrical part 215 locates spindle 217 whilst allowing relative rotation of the spindle 217 within the cylindrical part 215. A corresponding cylindrical part 219 is formed on the inside of end cap 213. When the end cap 213 is secured to the main body 201 by co-operation of the screw threads 209 and 211 the cylindrical part 219 locates the other end of the spindle 217 whilst allowing rotation of the spindle.

The end of the spindle 217 that is accommodated in the cylindrical part 219 of the end cap 213 includes a non-cylindrical recess 221 into which a correspondingly shaped protrusion 223 of gear 225 is inserted. When the protrusion 223 is inserted in the recess 221 relative rotation between the spindle 217 and the gear 225 is not permitted. Thus, rotation of the gear 225 (by external means, not shown) causes rotation of the spindle 217.

The side wall 207 of the main body 201 has an opening 227 formed therein around which an external flange 229 is formed. The flange 229 has a fluid port 31 formed therein, fluid for which is provided by conventional external means (not shown).

A suitable liner 233, which can be preformed as shown in Figure 16, is wound around the spindle 217. The liner 233 is attached at one end 235 to the spindle 233 (Figure 21). The other end 237 of the liner 233 passes along the internal surface of the flange 229 and is folded back and secured to the external surface of the flange 229 by fluid-tight seal 239. The flange 229 is inserted into the opening of the patient's anus 241, in use.

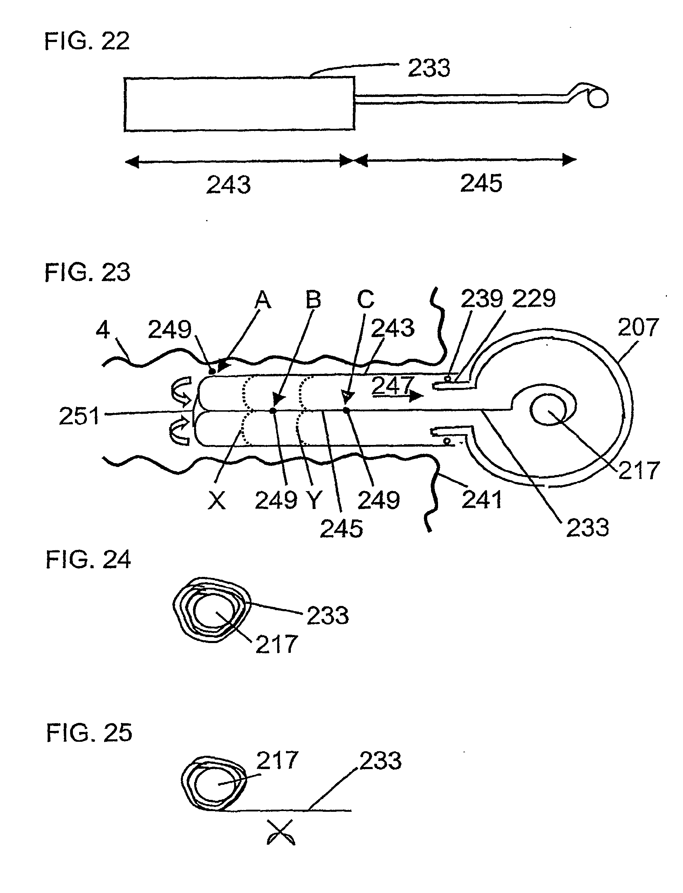

Figure 22 shows the liner 233 in the state after manufacture and before use. The liner may comprise polyurethane or latex, or any other suitable eversible material known to those skilled in the art. The liner includes a first portion 243 which is approximately 1.8m long and 30mm in diameter. The second portion 245 is also approximately 1.8m long but is of a smaller diameter. 1.8m is generally the maximum distance that a liner is inserted into the human colon. Of course, the length of portions 243 and 245 will be varied depending on the particular application. For example, if the patient is an animal, the lengths of portions 243 and 245 will be adjusted in accordance with the length of the animal's colon. It is preferable, although not essential, that portions 243 and 245 are of substantially equal length. The portion 243 performs the function of a swab. That is, it is intended to record a bacterial imprint of the surface of the colon. The surface of the portion 243 of the liner may be specially treated to make it particularly suitable for this purpose. It should be understood that it is quite possible to have the entire length of the liner 233 formed of a 30mm diameter swab portion, rather than having separate first 243 and second 245 parts. However, the arrangement illustrated in Figure 22 is preferable as it less wasteful of material.

As mentioned above, when it is intended to swab the patient's colon (i.e. to obtain a sample of material along the length of the colon), the flange 229 is inserted into the opening of the patient's anus. Fluid pressure is applied to the liner 233 via fluid port 231. The fluid causes the liner 233 to evert and travel up the colon in the direction of arrow 246 until the liner is everted sufficiently to line the required length of the colon, as shown in Figure 223. The required length will depend upon how much of the colon the physician wishes to swab. Generally, the liner 233 will evert until it reaches the patient's caecum. The liner's path along the patient's colon will be as that described in relation to the prior art liner described above, which was for facilitating a colonoscopy.

As the liner 233 is everted the spindle rotates within cylindrical parts 215 and 219 in order to provide further liner material as it is required. Fluid pressure within the liner 233 urges the liner against the inner surface of the colon. Bacteria on the inner surface of the colon are transferred to the surface of the liner 233 as the liner contacts the colon. When the liner is fully extended (Figure 23) the surface of the liner 233 is in contact with the entire inner surface of the colon that requires analysis. The surface of the liner 233 in contact with the internal surface of the patient's colon will correspond to the part 243 of the liner 233, as shown in Figure 3. Fluid pressure will cause the inner, opposite walls of the liner to press against each other generally along the central axis of the colon, thereby temporarily completely blocking the colon. When the liner 233 is fully extended within the colon the inner walls of the liner will correspond generally to the second part 245 of the liner as shown in Figure 22.

The liner 233 is withdrawn from the colon by engaging the protrusion 223 of the gear 225 in the recess 221 in the spindle 217. The gear 225 is rotated by external means, as discussed above. This rotation causes corresponding rotation of the spindle 217. This rotation will be in the opposite sense to the rotation of the spindle which occurred during eversion of the liner. The rotation of the spindle 217 will pull the liner back away from the patient's caecum, in the direction indicated by arrow 247. This causes the liner 233 to peal away from the internal surface of the colon. Figure 23 shows schematically bacteria 249 from the colon which have adhered to the surface of the liner 233. The bacteria 249 on the colon are transferred to the liner 233 at position A. As the spindle 217 is rotated this draws the liner in the direction of arrow 247, as mentioned above. The bacteria, 249, which are adhered to the liner 233 momentarily are located at the distal end of the liner before passing between the two touching inner walls of the liner, located generally along the central axis of the colon 241. As the liner is withdrawn from the colon the distal end 251 moves to the first position X indicated by a dashed line. When the distal end 251 of the liner is at position X, the bacteria 249 will have moved to position B. After further retraction of the liner 233, the distal end will be at position Y, and the bacteria 249 will be at position C.

During retraction of the liner 233 from the patient's colon, a continuous fluid pressure is maintained via port 231. The continuous fluid pressure may be lower than the pressure used to evert the liner 233 but should be sufficient to prevent the liner from folding during retraction.

The transfer of bacteria 249 to the liner 233 requires minimal rubbing action to transfer the depositions. When the liner 233 has been fully retracted (inverted) the seal 239 between the liner 233 and the flange 229 will be removed. The spindle 217 around which the full length of the liner 233 is coiled can be removed from the main body 201 by removal of the end cap 213. The removed spindle 217 and liner 233 (Figure 24) can then be sent away for analysis. For example, this analysis could comprise gradually uncoiling the liner 233 and cutting it into small sections, for example, of 10mm in length. The sections could be cultured to detect the bacteria present at each location along the liner. The information derived from each section provides an indication of the bacteria present at the corresponding location of the patient's colon. Hitherto, information about the location of bacteria within the colon has not been available. This might be particularly useful for medical research by clinicians and drug/food companies, and by patients who want to know the balance of their microflora.

In conditions that affect the bowel, such as irritable bowel syndrome or chronic constipation, a comparison between the normal population's bacterial microflora and the patient's could be made. Also, drug/food companies wanting to assess the effect of new medications/probiotics on bowel function could use the device.

The apparatus and method described produces a "bacterial map" of the bacteria present in a patient's colon. The apparatus and method could also be used for cytology. With this process cells may be flaked from the colon wall by vibration or other means of an abrasive surface. The cells can then be tested to see if they are cancerous.

A single walled liner is shown forming the swab. Alternatively, the structures of the first or second embodiments can be used to deploy a swab. In the first embodiment, the swab would correspond to the liner. In the second embodiment, the swab would correspond to the membrane 110.

A conventional liner/membrane could be modified for use on a swab by providing a friction or other non-smooth surface for the lining/membrane. The surface could be configured to perform the swab function.

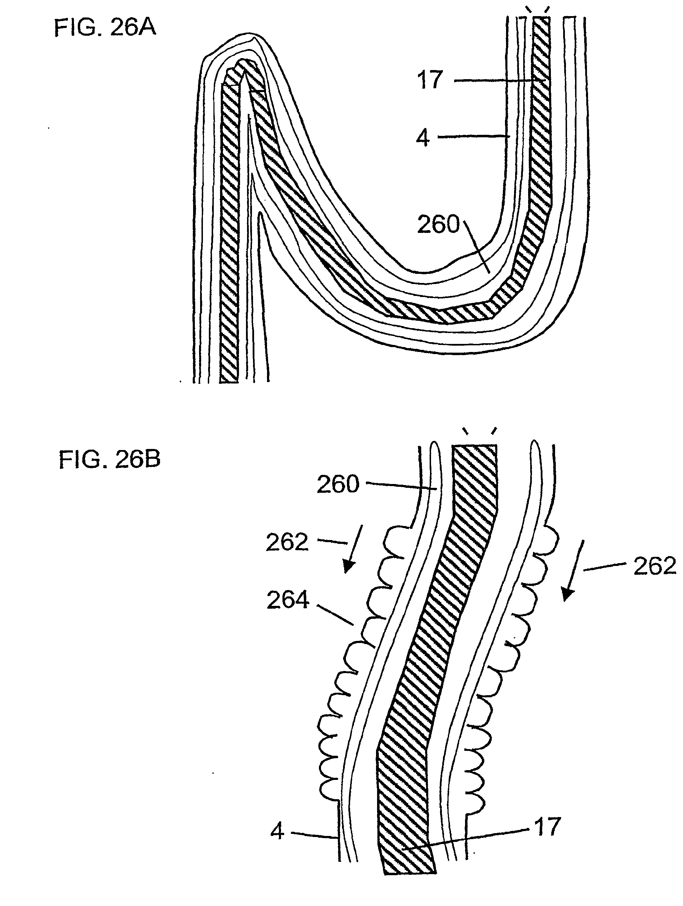

As described in relation to the prior art, looping of the colon 4 may occur as a result of applying forward pressure to an endoscope 17 when the required direction of movement of the endoscope 17 is in a direction different to the force applied. Looping of the colon is shown in Figure 26A. According to an embodiment of the present invention, the looped colon 4 may be straightened by pulsing the pressure within the membrane 260 so that the pressure is increased and then reduced, possibly repeatedly. This will cause the colon 4 walls to move along with the outside surface of the membrane 260, as shown by the arrows 262. The loop of the colon 4 will be corrected, the overall length of the looped portion reducing, resulting in "bunching" of the colon 4 material as shown at 264 in Figure 26B. The membrane 260 may be a conventional single walled liner, a double walled liner as in the first embodiment or a membrane/endoscope 17 arrangement as in the second embodiment.

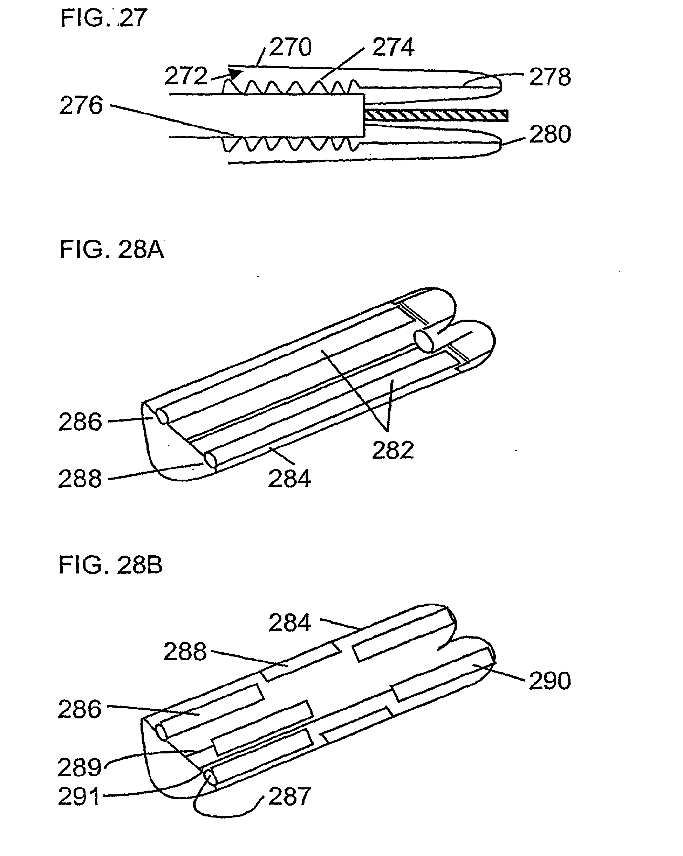

Figure 27 shows another example, not forming part of the claimed invention. In this embodiment a single-walled liner 270 is provided. The liner 270 is inflated by application of fluid pressure from fluid source 272. However, in contrast to a conventional single-walled liner, a semi-rigid support member 274 is mounted upon a storage member 276. The portion of the semi-rigid support member mounted upon the storage means 276 is in concertinered form, although it may be mounted in helical or other form. However, the arrangement is such that as the liner 270 everts material of the semi-rigid member 274 will be released from the storage means 276 so that the distal end 278 of the semi-rigid support member always contacts the distal end 280 of the liner 270. Thus, the semi-rigid support member 274 extends automatically as the liner 270 is everted. Such an arrangement may assist a single-walled liner negotiating curves within the colon.

Figures 28A and 28B show a modification to the design described in relation to Figure 5. However, in Figures 28A and 28B, instead of the fluid pockets extending throughout the length of the liner or membrane, the pockets extend for predetermined lengths and may have individually controlled fluid pressures to provide rigid structures to individual sections of the membrane or liner. In Figure 28A fluid pockets 282 are provided within the liner 284. The fluid pockets 282 are provided with fluid from a first supply 286, and the liner is provided with fluid from a second fluid supply 288. In Figure 28B the liner 284 includes a first set of fluid pockets 286, supplied by first fluid supply 287. A second set of fluid pockets 288 is supplied by fluid supply 289. A third set of fluid pockets 290 are supplied by fluid supply 291. Obviously, the number of fluid pockets in any one of these sets could be varied according to the requirements. The three sets of fluid pockets are longitudinally spaced apart from one another along the length of the liner 284. By having separate fluid supplies, the fluid pressure in the sets of fluid pockets can be controlled individually, such that the rigidity of the liner 284 can be varied along its length. Therefore, one portion of the liner 284 could be made rigid whilst other portions were flaccid. This is particularly advantageous for negotiating tight curves of the colon 4 during eversion.

During withdrawal of the liner it is desirable that the liner is progressively inverted. However when the endoscope is withdrawn around a 90° curve of the colon, this will tend to cause both sides of the liner (the side contacting the endoscope and the side contacting the colon) to be withdrawn - which is undesirable. Inflating a fluid pocket at the location of a severe curve will assist in proper inversion by maintaining the liner in position.

Although the sets of pockets in Figure 28A and 28B are shown to be spaced apart longitudinally, they could alternatively or additionally be circumferencially or vertically spaced apart.

The pockets could be formed by making a plurality of pockets extending along the entire length of the membrane - like the pockets 15 of the Figure 5. The pockets could then be closed-off at required intervals along their length to provide separate sub-pockets, for example, by the application of heat during manufacture. Each of the sub-pockets could be supplied with fluid by a small flexible pipe.

Alternatively, one of the fluid pockets 282 of Figure 28A could be modified to form a structure open at each end. If the pocket 282 was formed to be half the length of the fully everted liner, a surgical instrument or other semi-rigid member could be passed along the pocket to the distal end of the liner. Because the pocket is open at both ends, the surgical instrument or member does not need to puncture the pocket (as described in relation to Figure 5), and nor is an access valve 300 required (as described in relation to Figure 2A).

## Claims

1. Body cavity lining means having at least one fluid chamber (9) and at least one further means (23) for providing lining means rigidity, in use, **characterised in that** said at least one further means (23) for providing lining means rigidity comprises an eversible double walled portion defining a further fluid chamber (23).

2. Lining means as claimed in claim 1, wherein the further chamber (23) is divided into a plurality of pockets (15, 282, 286, 288. 290).

3. Lining means as claimed in claim 2, wherein means is provided for applying different fluid pressure to respective ones of the pockets (15, 282, 286, 288, 290).

4. Lining means as claimed in claim 2 or 3, wherein a pocket (15, 282, 286, 288, 290) is provided with valve means (300) for allowing the passage of apparatus therethrough.

5. Lining means as claimed in claim 2, 3 or 4, wherein respective ones of the pockets (15. 282. 286, 288. 290) are positioned at longitudinally spaced intervals along the length of the lining means.

6. Lining means as claimed in claim 1, 2, 3, 4 or 5, wherein the double walled portion (3) extends for substantially half the length of the lining means, the remaining length (1) of the lining means being single walled.

7. Lining means as claimed in any one of the preceding claims, wherein the at least one of said chambers (156) is formed with a curved configuration.

8. Lining means as claimed in any one of the preceding claims wherein the at least one of said chambers (160) is formed with a portion adapted to facilitate curvature of that portion.

9. Lining means as claimed in any one of the preceding claims, including valve means (172) mounted on one of said chambers (170) operable for resisting the passage of fluid from the lining means to the body cavity.

10. Lining means as claimed in claim 9, wherein the valve means (172) is operable to allow the passage of a member therethrough.

11. Lining means according to any one of the preceding claims, wherein at least one of the chambers (233) is adapted to carry bacteria or other cells from the body cavity.

12. Lining means according to any one of the preceding claims, including first means for controlling fluid pressure in said chamber (9) to cause said chamber (9) to evert and advance in said body cavity, and second means for controlling fluid pressure in said further fluid chamber (23).

13. Lining means according to claim 12, including means for controlling the application of fluid by said first and second fluid controlling means such that a member (17) can be inserted into said body cavity (4) as said chamber everts (9, 117).

14. Lining means according to claim 13, wherein the means for controlling the application of fluid by said first and second fluid controlling means is operable so as to enable removal of said member (17) without disturbing the position of the lining means such that an alternative instrument can be inserted through the lining means.

15. Lining means according to any one of the preceding claims, including a housing (30, 39, 40, 42, 116) for storing at least said chamber (9, 117) before eversion and allowing release of said chamber (9, 117) during eversion.

16. Lining means according to claim 15, wherein said housing (30, 39, 40, 42, 116) includes an extendible cover portion (44, 46) for accommodating the chamber material as it is withdrawn from said body cavity.

17. Lining means according to any one of the preceding claims, including an enlargeable part (146, 148) for frictionally engaging the body cavity.

18. Lining means according to claim 17, wherein the enlargeable part (146) is formed integrally with one of said chambers.

19. Lining means according to any one of the preceding claims, the lining means (156, 160) being adapted to tend to conform to the curvature of a predetermined body cavity type.

20. A method of lining a body cavity (4) of an inanimate object, including an excised or artificial human or animal body part, the method including providing lining means having two fluid chambers (9, 23, 117, 129); selectively applying fluid to a first of the chambers (9, 117) so as to cause said first chamber (9, 117) to evert and advance into said body cavity (4); and selectively applying fluid to a second of said chambers (23, 129), **characterised in that** the step of applying fluid to the second chamber (23,129) applies fluid to an eversible double walled portion.

21. A method as claimed in claim 20, including inserting a member (17) in said lining means such that eversion of the first chamber (9, 117) causes the member to advance in said body cavity (4).

22. A method as claimed in claim 20 or 21, including removing the member (17) from said lining means without disturbing the position of the lining means, such that an alternative instrument can be inserted through the lining means.

23. A method as claimed in claim 21 or 22, wherein the member (17) is retracted by reducing fluid pressure to the first chamber (9), the arrangement being such that the friction between the first chamber (9) and the member (17) is reduced whilst the fluid pressure in the second chamber (23) causes the liner to remain in position in the body cavity (4).

24. A method according to claim 20,21,22 or 23, including withdrawing the lining means from the body cavity (4), and analysing material deposited on the lining means.

25. A method according to any one of claims 20 to 24, wherein a deformable tube (142, 162) is positioned between the first and second chambers (117, 129).

26. A method according to any one of claims 20 to 25, wherein fluid is applied to one of said chambers (9, 23, 117, 129) to reduce friction between the second chamber (23, 117) and the first chamber (9, 129).

## Patentansprüche

1. Körperhöhlen-Auskleideeinrichtung, die wenigstens eine Fluidkammer (9) und wenigstens eine weitere Einrichtung (23) aufweist, die der Auskleideeinrichtung in Funktion Steifigkeit verleiht, **dadurch gekennzeichnet, dass** die wenigstens eine weitere Einrichtung (23), die der Ausleitungseinrichtung Steifigkeit verleiht, einen ausstülpbaren doppelwandigen Abschnitt umfasst, der eine weitere Fluidkammer (23) bildet.

2. Auskleideeinrichtung nach Anspruch 1, wobei die weitere Kammer (23) in eine Vielzahl von Taschen (15) unterteilt ist.

3. Auskleideeinrichtung nach Anspruch 2, wobei benachbarte Taschen, (15) in Fluidverbindung stehen und Fluid von Tasche zu Tasche gelangen lassen.

4. Auskleideeinrichtung nach Anspruch 2, wobei eine Einrichtung zum Ausüben von unterschiedlichem Fluiddruck auf entsprechende der Taschen (15) vorhanden ist.

5. Auskleideeinrichtung nach Anspruch 2 oder 4, wobei eine Tasche (15) mit einer Ventileinrichtung (30) versehen ist, die das Hindurchtreten von Vorrichtungen durch sie ermöglicht.

6. Auskleideeinrichtung nach Anspruch 2, 3, 4 oder 5, wobei entsprechende der Taschen in in Längsrichtung beabstandeten Intervallen entlang der Länge der Auskleideeinrichtung angeordnet sind.

7. Auskleideeinrichtung nach Anspruch 1, 2, 3, 4, 5 oder 6, wobei sich der doppelwandige Abschnitt (3) im Wesentlichen über die halbe Länge der Auskleideeinrichtung erstreckt und die verbleibende Länge (1) der Auskleideeinrichtung einwandig ist.

8. Auskleideeinrichtung nach einem der vorangehenden Ansprüche, wobei die wenigstens eine der Kammern (156) mit einer gekrümmten Form versehen ist.

9. Auskleideeinrichtung nach einem der vorangehenden Ansprüche, wobei die wenigstens eine der Kammern (160) mit einem Abschnitt versehen ist, der so eingerichtet ist, dass er Krümmung dieses Abschnitts erleichtert.

10. Auskleideeinrichtung nach einem der vorangehenden Ansprüche, die eine Ventileinrichtung (172) enthält, die an einer der Kammern (170) angebracht ist und in Funktion dem Hindurchtreten von Fluid von der Auskleideeinrichtung zu der Körperhöhle Widerstand entgegensetzt.

11. Auskleideeinrichtung nach Anspruch 10, wobei die Ventileinrichtung (172) so betrieben werden kann, dass sie das Hindurchtreten eines Elementes zulässt.

12. Auskleideeinrichtung nach einem der vorangehenden Ansprüche, wobei wenigstens eine der Kammern (233) so eingerichtet ist, dass sie Bakterien oder andere Zellen aus der Körperhöhle transportiert.

13. Auskleideeinrichtung nach einem der vorangehenden Ansprüche, die eine erste Einrichtung enthält, die Fluiddruck in der Kammer (9) steuert, um zu bewirken, dass die Kammer (9) umgestülpt wird und sich in der Körperhöhle vorwärtsbewegt, und eine zweite Einrichtung enthält, die Fluiddruck in der weiteren Fluidkammer (23) steuert.

14. Auskleideeinrichtung nach Anspruch 13, die eine Einrichtung enthält, die die Zufuhr von Fluid durch die erste und die zweite Fluid-Steuereinrichtung so steuert, dass ein Element (17) in die Körperhöhle (4) eingeführt werden kann, wenn die Kammer (9, 117) ausgestülpt wird.

15. Auskleideeinrichtung nach Anspruch 14, wobei die Einrichtung, die die Zufuhr von Fluid durch die erste und die zweite Fluid-Steuereinrichtung steuert, so betätigt werden kann, dass das Entfernen des Elementes (17) ohne Störung der Position der Auskleideeinrichtung ermöglicht wird, so dass ein alternatives Instrument über die Auskleideeinrichtung eingeführt werden kann.

16. Auskleideeinrichtung nach einem der vorangehenden Ansprüche, die ein Gehäuse (30, 39, 40, 42, 116) zum Aufbewahren der wenigstens einen Kammer (9, 117) vor dem Ausstülpen enthält, das Freigabe der Kammer (9, 117) während des Ausstülpens ermöglicht.

17. Auskleideeinrichtung nach Anspruch 16, wobei das Gehäuse (30, 39, 40, 42, 116) einen dehnbaren Abdeckabschnitt (44, 46) enthält, das das Kammermaterial aufnimmt, wenn es aus der Körperhöhle herausgezogen wird.

18. Auskleideeinrichtung nach einem der vorangehenden Ansprüche, die einen vergrößerbaren Teil (146, 148) enthält, der in Reibungseingriff mit der Körperhöhle kommt.

19. Auskleideeinrichtung nach Anspruch 18, wobei der vergrößerbare Teil (1456) integral mit einer der Kammern ausgebildet ist.

20. Auskleideeinrichtung nach einem der vorangehenden Ansprüche, wobei die Auskleideeinrichtung (156, 160) so eingerichtet ist, dass sie sich an die Krümmung eines vorgegebenen Typs von Körperhöhle anpasst.

21. Verfahren zum Auskleiden einer Körperhöhle (4) eines unbelebten Objektes, einschließlich eines herausgeschnittenen oder künstlichen menschlichen oder tierischen Körperteils, wobei das Verfahren Bereitstellen einer Auskleideeinrichtung mit zwei Fluidkammern (9, 23, 117, 129), selektives Zuführen von Fluid zu einer ersten der Kammern (9, 117), um zu bewirken, dass die erste Kammer ausgestülpt wird und sich in die Körperhöhle (4) hinein vorwärtsbewegt; und selektives Zuführen von Fluid zu einer zweiten der Kammern (23, 129) einschließt, **dadurch gekennzeichnet, dass** der Schritt des Zuführens von Fluid zu der zweiten Kammer (23, 129) einem ausstülpbaren doppelwandigen Abschnitt Fluid zuführt.

22. Verfahren nach Anspruch 21, das das Einführen eines Elementes (17) in die Auskleideeinrichtung einschließt, so dass Ausstülpen der ersten Kammer (9, 117) bewirkt, dass sich das Element in die Körperhöhle (4) hinein vorwärtsbewegt.

23. Verfahren nach Anspruch 21 oder 22, das Entfernen des Elementes (17) aus der Auskleideeinrichtung ohne Störung der Position der Auskleideeinrichtung einschließt, so dass ein alternatives Instrument über die Ausleitungseinrichtung eingeführt werden kann.

24. Verfahren nach Anspruch 22 oder 23, wobei das Element (17) eingezogen wird, indem Fluiddruck auf die erste Kammer (9) reduziert wird, und die Anordnung so ist, dass die Reibung zwischen der ersten Kammer (9) und dem Element (17) reduziert wird, während der Fluiddruck in der zweiten Kammer (23) bewirkt, dass die Auskleidung in der Körperhöhle (4) in Position bleibt.

25. Verfahren nach Anspruch 21, 22, 23 oder 24, das Herausziehen der Auskleideeinrichtung aus dem Körperhohlraum (4) und Analysieren von an der Auskleideeinrichtung abgelagertem Material einschließt.

26. Verfahren nach einem der Ansprüche 21 bis 25, wobei eine verformbare Röhre (142, 152) zwischen der ersten und der zweiten Kammer (117, 129) angeordnet ist.

27. Verfahren nach einem der Ansprüche 21 bis 26, wobei Fluid einer der Kammern (9, 23, 117, 129) zugeführt wird, um Reibung zwischen der zweiten Kammer (21, 117) und der ersten Kammer (9, 129) zu reduzieren.

28. Verfahren nach einem der Ansprüche 21 bis 27, wobei die zweite Kammer (23) in eine Vielzahl von Taschen (15) unterteilt ist und benachbarte Taschen in Fluidverbindung stehen, und mit dem Schritt des Zuführens von Fluid den Taschen Fluid zugeführt wird und das Fluid von Tasche zu Tasche gelangt.

## Revendications

1. Moyens de revêtement de cavité corporelle comprenant au moins une chambre de fluide (9) et au moins d'autres moyens (23) pour fournir la rigidité des moyens de revêtement, à l'usage, **caractérisés en ce que** ledit au moins un moyen supplémentaire (23) pour fournir la rigidité des moyens de revêtement comprend une partie à double paroi pouvant se renverser définissant une autre chambre de fluide (23).

2. Moyens de revêtement selon la revendication 1, dans lesquels la chambre supplémentaire (23) est divisée en une pluralité de poches (15).

3. Moyens de revêtement selon la revendication 2, dans lesquels les poches (15) adjacentes sont en communication de fluide et permettent au fluide de passer d'une poche à l'autre.

4. Moyens de revêtement selon la revendication 2, dans lesquels on propose des moyens pour appliquer une pression de fluide différente sur les poches respectives des poches (15).

5. Moyens de revêtement selon la revendication 2 ou 4, dans lesquels une poche (15) est dotée de moyens formant clapet (300) pour permettre le passage de l'appareil à travers celle-ci.

6. Moyens de revêtement selon la revendication 2, 3, 4 ou 5, dans lesquels les poches respectives des poches (15) sont positionnées à des intervalles longitudinalement espacés le long de la longueur des moyens de revêtement.

7. Moyens de revêtement selon la revendication 1, 2, 3, 4, 5 ou 6, dans lesquels la partie à double paroi (3) s'étend sensiblement sur la moitié de la longueur des moyens de revêtement, la longueur (1) restante des moyens de revêtement étant à paroi unique.

8. Moyens de revêtement selon l'une quelconque des revendications précédentes, dans lesquels la au moins une chambre desdites chambres (156) est formée avec une configuration incurvée.

9. Moyens de revêtement selon l'une quelconque des revendications précédentes, dans lesquels la au moins une chambre desdites chambres (160) est formée avec une partie adaptée pour faciliter la courbure de cette partie.

10. Moyens de revêtement selon l'une quelconque des revendications précédentes, comprenant des moyens formant clapet (172) montés sur l'une desdites chambres (170) pouvant fonctionner pour résister au passage du fluide des moyens de revêtement à la cavité corporelle.

11. Moyens de revêtement selon la revendication 10, dans lesquels les moyens formant clapet (172) peuvent fonctionner pour permettre le passage d'un élément à travers ceux-ci.

12. Moyens de revêtement selon l'une quelconque des revendications précédentes, dans lesquels au moins l'une des chambres (233) est adaptée pour transporter les bactéries ou d'autres cellules depuis la cavité corporelle.

13. Moyens de revêtement selon l'une quelconque des revendications précédentes, comprenant des premiers moyens pour réguler la pression de fluide dans ladite chambre (9) afin d'amener ladite chambre (9) à se renverser et à avancer dans ladite cavité corporelle, et des seconds moyens pour réguler la pression de fluide dans ladite chambre de fluide supplémentaire (23).

14. Moyens de revêtement selon la revendication 13, comprenant des moyens pour réguler l'application du fluide par lesdits premiers et seconds moyens de régulation de fluide de sorte qu'un élément (17) peut être inséré dans ladite cavité corporelle (4) lorsque ladite chambre (9, 117) se renverse.

15. Moyens de revêtement selon la revendication 14, dans lesquels les moyens pour réguler l'application de fluide par lesdits premiers et seconds moyens de régulation de fluide peuvent fonctionner afin de permettre le retrait dudit élément (17) sans déranger la position des moyens de revêtement de sorte qu'un instrument en variante peut être inséré dans les moyens de revêtement.

16. Moyens de revêtement selon l'une quelconque des revendications précédentes, comprenant un boîtier (30, 39, 40, 42, 116) pour stocker au moins ladite chambre (9, 117) avant le renversement et permettre le dégagement de ladite chambre (9, 117) pendant le renversement.

17. Moyens de revêtement selon la revendication 16, dans lesquels ledit boîtier (30, 39, 40, 42, 116) comprend une partie de couvercle extensible (44, 46) pour recevoir le matériau de chambre lorsqu'il est retiré de ladite cavité corporelle.

18. Moyens de revêtement selon l'une quelconque des revendications précédentes, comprenant une partie pouvant être élargie (146, 148) pour mettre en prise par frottement la cavité corporelle.

19. Moyens de revêtement selon la revendication 18, dans lesquels la partie pouvant être élargie (146) est formée de manière solidaire avec l'une desdites chambres.

20. Moyens de revêtement selon l'une quelconque des revendications précédentes, les moyens de revêtement (156, 160) étant adaptés pour avoir tendance à se conformer à la courbure d'un type de cavité corporelle prédéterminé.

21. Procédé permettant de recouvrir une cavité corporelle (4) d'un objet inanimé, comprenant une partie corporelle animale ou humaine excisée ou artificielle, le procédé comprend les étapes consistant à prévoir des moyens de revêtement ayant deux chambres de fluide (9, 23, 117, 129) ; appliquer sélectivement le fluide sur une première chambre des chambres (9, 117) afin d'amener ladite première chambre (9, 117) à se renverser et à avancer dans ladite cavité corporelle (4) ; et appliquer sélectivement le fluide sur une seconde chambre desdites chambres (23, 129), **caractérisé en ce que** l'étape consistant à appliquer le fluide sur la seconde chambre (23, 129) applique le fluide sur une partie à double paroi pouvant se renverser.

22. Procédé selon la revendication 21, comprenant l'étape consistant à insérer un élément (17) dans lesdits moyens de revêtement de sorte que le renversement de la première chambre (9, 117) amène l'élément à avancer dans ladite cavité corporelle (4).

23. Procédé selon la revendication 21 ou 22, comprenant l'étape consistant à retirer l'élément (17) desdites moyens de revêtement sans perturber la position des moyens de revêtement, de sorte qu'un instrument en variante peut être inséré dans les moyens de revêtement.

24. Procédé selon la revendication 22 ou 23, dans lequel l'élément (17) est rétracté en réduisant la pression de fluide sur la première chambre (9), l'agencement étant tel que le frottement entre la première chambre (9) et l'élément (17) est réduit, alors que la pression de fluide dans la seconde chambre (23) oblige le revêtement à rester en position dans la cavité corporelle (4).

25. Procédé selon la revendication 21, 22, 23 ou 24, comprenant les étapes consistant à retirer les moyens de revêtement de la cavité corporelle (4) et à analyser le matériau déposé sur les moyens de revêtement.

26. Procédé selon l'une quelconque des revendications 21 à 25, dans lequel un tube déformable (142, 152) est positionné entre les première et seconde chambres (117, 129).

27. Procédé selon l'une quelconque des revendications 21 à 26, dans lequel le fluide est appliqué sur l'une desdites chambres (9, 23, 117, 129) pour réduire le frottement entre la seconde chambre (23, 117) et la première chambre (9, 129).

28. Procédé selon l'une quelconque des revendications 21 à 27, dans lequel la seconde chambre (23) est divisée en une pluralité de poches (15), dans lequel les poches adjacentes sont en communication de fluide et dans lequel l'étape consistant à appliquer du fluide, applique le fluide sur les poches et le fluide passe d'une poche à l'autre.
